# EUROPEAN PATENT SPECIFICATION

(11) **EP 1 964 546 B1**
(45) Date of publication and mention of the grant of the patent: **26.10.2016**
(21) Application number: 06833085.1
(22) Date of filing: 21.11.2006
(51) Int. Cl.: A61K 8/60, A61K 8/99, A61Q 19/00

(54) **SKIN CARE COSMETIC AND SKIN AND AGENT FOR PREVENTING SKIN ROUGHNESS CONTAINING BIOSURFACTANTS**
TENSIDE ENTHALTENDES HAUTPFLEGEMITTEL UND MITTEL ZUR VERHINDERUNG VON RAUHER HAUT
PRODUIT COSMÉTIQUE DE SOIN DE LA PEAU ET AGENT SERVANT À EMPÊCHER LA PEAU DE DEVENIR RÊCHE CONTENANT DES BIOTENSIOACTIFS

(30) Priority: 25.11.2005 JP 2005340902; 22.06.2006 JP 2006172238
(43) Date of publication of application: 03.09.2008
(73) Proprietor: Toyobo Co., Ltd., Osaka-shi Osaka 530-8230 (JP); National Institute of Advanced Industrial Science and Technology, Tokyo 100-8921 (JP)
(72) Inventor: KITAGAWA, Masaru, Otsu-shi Shiga 520-0292 (JP); SUZUKI, Michiko, Otsu-shi Shiga 520-0292 (JP); YAMAMOTO, Shuhei, Otsu-shi Shiga 520-0292 (JP); SOGABE, Atsushi, Otsu-shi Shiga 520-0292 (JP); KITAMOTO, Dai, Tsukuba-shi Ibaraki 3058565 (JP); IMURA, Tomohiro, Tsukuba-shi Ibaraki 3058565 (JP); FUKUOKA, Tokuma, Tsukuba-shi Ibaraki 3058565 (JP); MORITA, Tomotake, Tsukuba-shi Ibaraki 3058565 (JP)
(74) Representative: Müller-Boré & Partner Patentanwälte PartG mbB
(86) International application number: PCT/JP2006/323239
(87) International publication number: WO 2007/060956

(56) References cited:
- WO-A1-2004/020647
- JP-A- 2002 101 847
- JP-A- 2003 261 424
- JP-A- 2005 068 015
- JP-A- 2005 104 837
- KITAMOTO D ET AL: "MICROBIAL CONVERSION OF N-ALKANES INTO GLYCOLIPID BIOSURFACTANTS, MANNOSYLERYTHRITOL LIPIDS, BY PSEUDOZYMA (CANDIDA ANTARCTICA)", BIOTECHNOLOGY LETTERS, SPRINGER, vol. 23, no. 20, October 2001 (2001-10), pages 1709-1714, XP008014848, ISSN: 0141-5492, DOI: 10.1023/A:1012464717259
- KITAMOTO D. ET AL.: 'Functions and potential applications of glycolipid biosurfactants - from energy-saving materials to gene delivery carriers' JOURNAL OF BIOSCIENCE AND BIOENGINEERING vol. 94, no. 3, 2002, pages 187 - 201, XP008014846

## Description

### TECHNICAL FIELD

The present invention relates to the use of a biosurfactant or a premixed product thereof for skin care/skin roughness improvement, in particular the use of a biosurfactant as a cosmetic, and further skin care/skin roughness-improvement cosmetics containing the biosurfactant or the premixed product thereof. The biosurfactant is a mannosylerythritol lipid (hereinafter referred to as a "MEL"), e.g., mannosylerythritol lipid A (hereinafter referred to as "MEL-A"), mannosylerythritol lipid B (hereinafter referred to as "MEL-B") or mannosylerythritol lipid C (hereinafter referred to as "MEL-C"); or a mannosylmannitol lipid (hereinafter referred to as a "MML").

### BACKGROUND ART

Rough skin refers to skin in a dry state, on which the exfoliation of corneocytes is observed. This type of rough skin develops due to an elution of intercorneocyte lipids such as cholesterol, ceramide and fatty acids, the formation failure of a horny layer permeation barrier because of the denaturation of the corneocytes, and the disturbance of the proliferation/keratinization balance in epidermal cells attributable to ultraviolet light and detergents. Supplying intercorneocyte lipid components or synthetic intercorneocyte lipids analogous thereto for the purpose of preventing or treating aforementioned rough skin has been studied.

Lamella granules biosynthesized in cells in a spinous layer and a granular layer are released in intercellular space just under the horny layer, extend to take a lamella structure and spread in the intercellular space to produce the aforementioned intercorneocyte lipids. The lamella granules are composed of glycosylceramide, cholesterol, ceramide, phospholipids and the like; however, glycosylceramide is rarely included in the intercorneocyte lipids. That is, it is believed that glycosylceramide in the lamella granules is hydrolyzed with β-glucocerebrosidase and converted into ceramide, and that this ceramide takes the lamella structure, resulting in improved formation of the horny layer permeation barrier as the intercorneocyte lipid acts as a barrier to prevent rough skin. It has been reported that a ceramide supplement is effective against skin roughness due to a washing agent, and is highly effective for the improvement of skin roughness (Non-Patent Literature 1).

An extraction solution from a plant is composed mainly of glycosylceramide, but is not yet a satisfactory alternative to ceramide. During its synthesis, there are many reaction steps, and the cost to produce it on a large scale is high.

MEL is a yeast-produced, natural surfactant whose various physiological actions have been previously reported (Non-Patent Literature 2). Mannosylmannitol lipids (MML) obtained by replacing erythritol with mannitol have been discovered recently (Patent Document 1). Their use in external preparations and cosmetics, as anti-inflammatory agents and anti-allergy agents (Patent Document 2), as baldness remedies and hair growth drugs (Patent Document 3), as well as their antibacterial actions (Patent Document 4) and surface tension lowering actions (Patent Document 5) have been recognized; however, the use of MEL as an alternative to ceramide effective for improving skin roughness has been unknown.
Patent Document 1: JP 2005-104837-A
Patent Document 2: JP 2005-68015-A
Patent Document 3: JP 2003-261424-A
Patent Document 4: JP Sho-57-145896-A
Patent Document 5: JP Sho-61-205450
Non-Patent Literature 1: Hihu to Biyoh (Skin and Beauty) 36:210, 2004
Non-Patent Literature 2: Journal of Bioscience and Bioengineering 94:187, 2002

### DISCLOSURE OF INVENTION

### PROBLEM TO BE SOLVED BY THE INVENTION

Ceramide is used in cosmetics as a component useful for skin roughness, but because a synthesized product or an extracted product made from plants is expensive, only a small amount of ceramide is currently used. It is a major object of the present invention to provide an external preparation for skin using a biosurfactant produced by a microorganism as an alternative to ceramide. That is, the present invention provides the biosurfactant by which the formation of the horny layer permeation barrier is improved, thereby achieving the improvement of rough skin and that is an easily available lipid component, as an external preparation for the skin.

### MEANS FOR SOLVING THE PROBLEMS

As a result of an extensive study to overcome the above problems, it was found that the biosurfactant acts as an alternative to ceramide after adding the biosurfactant to a skin roughness model made by a three-dimensional skin model defatted with sodium lauryl sulfate (SDS); the usefulness of the biosurfactant was further proved by applying it on a rough skin site produced by treating human skin with SDS. That is, it was discovered that the biosurfactant could be used not only as an emulsifier, but could also be used in place of ceramide; thus, the present invention was completed.

The present invention relates to the following skin care cosmetic and skin roughness-improving agent.
(1) A skin care cosmetic comprising at least one biosurfactant.
(2) The skin care cosmetic according to (1) for improving skin roughness.
(3) The skin care cosmetic according to (1) for improving skin roughness by an action of a surfactant.
(4) The skin care cosmetic according to (1), wherein the biosurfactant is a mannosylerythritol lipid (MEL) and/or a mannosylmannitol lipid (MML).
(5) The skin care cosmetic according to (1), wherein the biosurfactant is at least one selected from the group consisting of mannosylerythritol lipid A (MEL-A), mannosylerythritol lipid B (MEL-B) and mannosylerythritol lipid C (MEL-C).
(6) The skin care cosmetic according to (1), wherein the biosurfactant is the mannosylerythritol lipid B (MEL-B).
(7) The skin care cosmetic according to (1), wherein the biosurfactant is the mannosylerythritol lipid C (MEL-C).
(8) The skin care cosmetic according to (1), wherein the biosurfactant is the mannosylerythritol lipid A (MEL-A).
(9) A skin roughness-improving agent consisting of at least one biosurfactant.

### EFFECT OF THE INVENTION

It has been found in the present invention that MELs such as MEL-A, MEL-B and MEL-C, and MML, which are the biosurfactants produced by microorganisms, can be used in place of ceramide for skin care and skin roughness improvement. The biosurfactant can be produced on a large scale by culturing the microorganism. By the use thereof of the ceramide alternative, skin roughness improvement/skin care action and an emulsifying action can be expected. Thus, it is possible to obtain an external preparation for the skin that is effective for improving skin roughness. In particular, MEL-B and MEL-C are highly hydrophilic, and can make stable emulsifiers. The biosurfactant may be used as a premixed product.

MELs are preferable because MELs may be combined in cosmetics and external preparations for the skin by dissolution in an oil base or in an oil-soluble component, and can be prepared as an aqueous solution (e.g., skin lotion, moisturizing liquid) by incorporating in the MEL a liposome, which is excellent for incorporation into the skin. The liposome can be used in a form other than in an aqueous solution. It is not necessary for all of the MELs to be formed into liposomes, and MELs as liposomes may be mixed with lamella-shaped MELs or MELs with simple bodies.

The biosurfactant is particularly useful as a skin care cosmetic and as a cosmetic for the improvement of skin roughness.

The biosurfactant is useful as the component combined with the cosmetics for washing because the biosurfactant has a detergent property in addition to its skin roughness improvement/skin care action.

Accordingly, the present invention concerns a use of the above-mentioned bio-surfactants mannosylerythritol (MML) and/or mannosylmannitol lipid (MML)for improving skin roughness as defined in the claims.

### BRIEF DESCRIPTION OF DRAWINGS

FIG. 1 is a graph showing the effects of MEL by viability (absorbance of formazan) in a skin roughness model made by using a three-dimensional skin model and treatment with SDS;
FIG. 2 is a graph showing the recovery of moisture in the horny layer when an MEL-A-containing cream is applied on skin on an upper portion of a human arm roughened by treatment with SDS;
FIG. 3 is a graph showing the effects of MEL-A, MEL (OL) and MEL (MY) by the viability (absorbance of formazan) in the skin roughness model made by using the three-dimensional skin model and treatment with SDS; MEL-A = MEL produced by culturing with soybean oil; MEL (MY) = MEL produced by culturing with methyl myristate and MEL-A (OL) = MEL produced by culturing with olive oil;

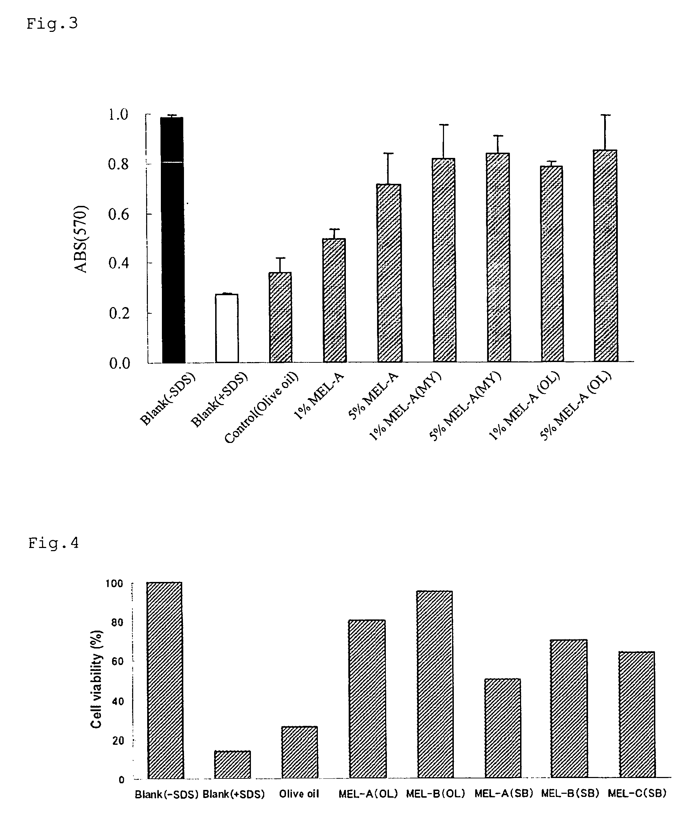

FIG. 4 is a graph showing the effects of MEL-B and MEL-C by the viability (absorbance of formazan) in the skin roughness model made by using the three-dimensional skin model and treating with SDS; MEL-B is the increased skin roughness improvement over MEL-A; in the figure, MEL-A (OL) is MEL-A produced using olive oil as a raw material, MEL-B (OL) is MEL-B produced using olive oil as a raw material, MEL-A (SB) is MEL-A produced using soybean oil as a raw material, MEL-B (SB) is MEL-B produced using soybean oil as a raw material, and MEL-C (SB) is MEL-C produced using soybean oil as a raw material;
FIG. 5 is a graph showing the recovery effects of the moisture contents in the horny layer when a MEL-B-containing cream was applied on skin on an upper portion of a human's arm roughened by treatment with SDS;
FIG. 6 is a graph showing the dispersion stability of MEL-B and MEL-C. MEL-B and MEL-C are more excellent than MEL-A in water dispersion stability; there is little observable change in turbidity because a stable suspension state is maintained even after 6 hours; and
FIG. 7 is a graph showing the results when a MEL-B liposome aqueous solution and a MEL-B suspension are subjected to a skin roughness test using the three-dimensional skin model, and the effects are examined.

### BEST MODES FOR CARRYING OUT THE INVENTION

The "biosurfactant" herein is a mannosylerythritol lipid (MEL) or mannosylmannitol lipid (MML).

The "premixed product" is one in which a dispersant is added in addition to the functional material, or that is diluted with a solvent to be easily used upon manufacturing the cosmetics. The biosurfactant of the present invention may be provided in the form of the premixed product, in which the dispersant and the solvent have been mixed as a cosmetic or a cosmetic additive for skin roughness improvement/skin care.

Ceramide is a sphingolipid that occupies about 50% of the intercellular lipids in the horny layer. Ceramide derived from bovine brain was often used previously, but, since the spread of mad cow disease, ceramide derived from plants has been required for cosmetics.

Ceramide-like actions are the actions in ceramide, the major intercellular component in the horny layer of the skin, that improve reduced skin tone and cosmetic fitness. The biosurfactant alone has ceramide-like skin roughness improvement/skin care actions; the effects of the biosurfactant can be improved by combining it with ceramide.

One advantage of a biosurfactant having ceramide-like actions is that it can be easily produced on a large scale and used as an emulsifier. Therefore, the biosurfactant of the present invention is more versatile than existing ceramide.

As the biosurfactant, mannosylerythritol lipid (MEL) or mannosylmannitol lipid (MML) are used. These form a lamella structure. Among these biosurfactants, MEL-A, MEL-B or MEL-C are preferable, and MEL-B or MEL-C are particularly preferable.

In one embodiment of the present invention, preferable MELs includes three types of MEL-A represented by the formula (I), MEL-B represented by the formula (II) and MEL-C represented by the formula (III); these may be used alone or in combination. MEL-A represented by the formula (I) and MEL-B represented by the formula (II) are more preferable, and MEL-B represented by the formula (II) is the most preferable.

MML is represented by the formula (IV) (in the formula, either or both of the acetyl groups at positions 4 and 6 in mannose may be substituted with hydroxyl group(s)).

R¹ and R² each have 1 to 19 carbon atoms, preferably 1 to 17, and more preferably 7 to 15.

R¹ and R² may be the same or different, and include C₁ to C₁₉ alkyl groups, C₂ to C₁₉ alkenyl groups, C₅ to C₁₉ alkadienyl groups and C₈ to C₁₉ alkatrienyl groups.

Preferable groups as R¹ and R² in the formulae (I), (II), (III) and (IV) include C₁ to C₁₉ alkyl groups such as CH₃(CH₂)₆, CH₃(CH₂)₈, CH₃(CH₂) ₁₀CH₃(CH₂)₁₂, CH₃(CH₂)₁₄, CH₃(CH₂)₁₆ and CH₃(CH₂)₁₈. MEL of the formula (I), (II) or (III) and MML of the formula (IV) in which R¹ and R² are C₁ to C₁₉ alkyl groups can be obtained by adding the raw material, e.g., a saturated carboxylic acid or unsaturated monocarboxylic acid such as formic acid, acetic acid, propionic acid, butyric acid, valeric acid, lauric acid, myristic acid, palmitic acid, stearic acid, oleic acid and palmitoleic acid or an ester thereof (monoalkyl esters, mono-, di-, triglyceride, or fats and oils including these saturated fatty acids or unsaturated monofatty acids) to the medium. When an unsaturated carboxylic acid such as oleic acid or palmitoleic acid is used as the raw material, the unsaturated carboxylic acid or a product during β-oxidation thereof is introduced, and thus the alkyl group becomes a major component and the alkenyl group becomes a minor component as R¹ and R². Likewise, the MEL of the formula (I), (II) or (III) and the MML of the formula (IV) in which R¹ and R² are C₂ to C₁₉ alkenyl groups, C₅ to C₁₉ alkadienyl groups or C₈ to C₁₉ alkatrienyl groups, can be obtained by adding the raw material, e.g., linoleic acid, linolenic acid, arachidonic acid, EPA, DHA having two or more double bonds or the ester thereof (monoalkyl ester, mono-, di-, triglyceride, or fats and oils including highly unsaturated fatty acids thereof) to the medium. For example, when linoleic acid is used as the raw material, the alkenyl group becomes the major component and the alkadienyl group becomes the minor component as R¹ and R². When linolenic acid is used as the raw material, the alkadienyl group becomes the major component and the alkenyl group or the alkatrienyl group becomes the minor component as R¹ and R².

These biosurfactants may be used alone, or two or more biosurfactants may be used in combination.

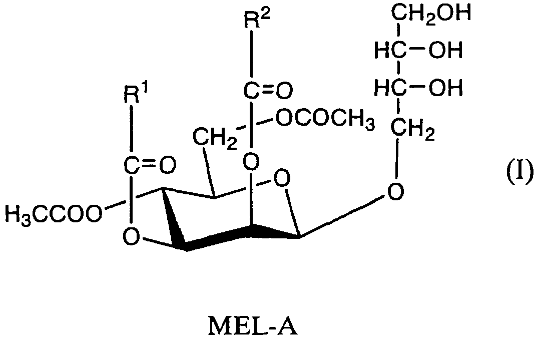

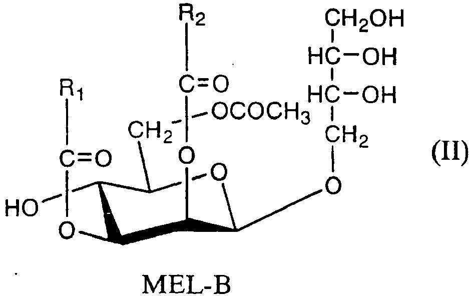

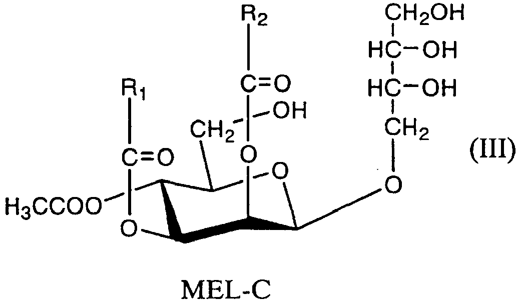

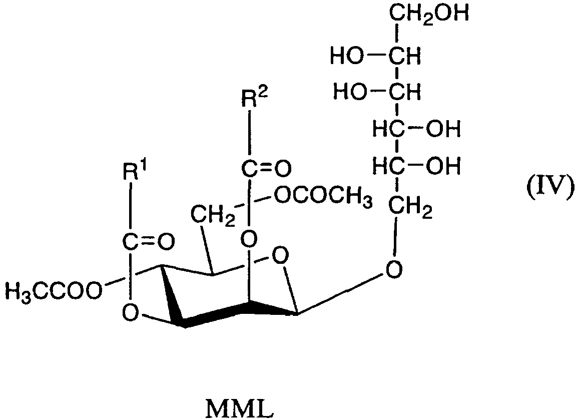

In the formulae (I) to (IV), R¹ and R² may be the same or different and represent hydrogen atoms; straight or branched C₁ to C₁₉, preferably C₁ to C₁₇, and more preferably C₇ to C₁₅ alkyl groups; straight or branched C₂ to C₁₉, preferably C₂ to C₁₇, and more preferably C₇ to C₁₅ alkenyl groups; straight or branched C₅ to C₁₉, preferably C₅ to C₁₇, and more preferably C₇ to C₁₅ alkadienyl groups; or straight or branched C₈ to C₁₉, preferably C₈ to C₁₇, and more preferably C₈ to C₁₅ alkatrienyl groups.

The method for producing the biosurfactant is not particularly limited, and a fermentation method using a microorganism could be optionally selected. For example, MELs (MEL-A, MEL-B and MEL-C) can be produced by culturing *Pseudozyma antarctica* NBRC 10736 according to standard methods. As the microorganism, *Pseudozyma antarctica, Pseudozyma sp.* and the like can be used. It is a well-known fact that a MEL mixture can be easily yielded from any microorganism. The MEL mixture can be purified using silica gel chromatography to isolate MEL-A, MEL-B and MEL-C. *Pseudozyma antarctica* and *Pseudozyma tsukubaensis* are known as the microorganisms that produce MEL-B, and these microorganisms may be used. *Pseudozyma hubeiensis* is known as the microorganism that produces MEL-C, and this microorganism may be used. The microorganism with an ability to produce MEL is not particularly limited, and can be appropriately selected depending on the purpose.

As the medium for fermentation to produce the biosurfactant, it is possible to use a common medium composed of N sources such as yeast extracts and peptone, C sources such as glucose and fructose, inorganic salts such as sodium nitrate, dipotassium hydrogen phosphate and magnesium sulfate heptahydrate, and those in which one or two or more nonaqueous substrates, e.g., fats and oils such as olive oil, soybean oil, sunflower oil, corn oil, canola oil and coconut oil, and hydrocarbons such as liquid paraffin and tetradecane, are added thereto can be used.

Fermentation conditions such as pH value, temperature and time period can be optionally set, and a culture medium after the fermentation can be directly used as the biosurfactant of the present invention. Optional manipulations such as filtration, centrifugation, extraction, purification and sterilization can be applied to the culture medium after fermentation, if necessary, in the range in which the essence of the present invention is not impaired, and it is still possible to dilute, concentrate and dry the resulting extract.

Plant fats and oils used as raw materials are not particularly limited, and can be appropriately selected depending on the purpose; these can include soybean oil, rapeseed oil, corn oil, peanut oil, cotton seed oil, safflower oil, sesame oil, olive oil and palm oil. Among them, soybean oil is particularly preferable in terms of enhancing production efficiency (produced amount, production speed and yield). These may be used alone or in combinations of two or more.

The inorganic nitrogen source is not particularly limited, and can be appropriately selected depending on the purpose; examples include ammonium nitrate, urea, sodium nitrate, ammonium chloride and ammonium sulfate.

The methods for collecting and purifying the biosurfactant are not particularly limited, and can be appropriately selected depending on the purpose; for example, the biosurfactant can be collected by centrifuging the culture medium to collect an oil content and extracting with an organic solvent such as ethyl acetate to concentrate.

As the solvent for the extraction, a mixed liquid obtained by mixing water with an organic solvent such as alcohols (e.g., lower alcohols such as methanol, absolute ethanol and ethanol, or polyvalent alcohols such as propylene glycol and 1,3-butylene glycol), ketones such as acetone, diethyl ether, dioxane, acetonitrile, esters such as ethyl acetate, xylene, benzene, and chloroform can be used alone or in combinations of two or more, and those obtained by combining respective solvent extracts can also be used.

The method for the extraction is not particularly limited. Typically, the extraction may be performed at temperatures ranging from ambient temperature to a boiling point of the solvent under an atmospheric pressure. After the extraction, the extract may be absorbed, decolorized or purified using the filtration or the ion exchange resin to make a solution, paste, gel or powder. In many cases, the resulting product can be directly used, but if necessary, a purification treatment such as a deodorant treatment or decoloration may be given thereto in the range in which the product's effectiveness is not affected. As a deodorant procedure and a decoloration procedure, an activated charcoal column can be used, and an ordinary procedure generally applied to an extracted substance can be optionally selected and performed. If necessary, a high-purity biosurfactant can be obtained by purification using a silica gel column.

As the biosurfactant, MEL-A, MEL-B and MEL-C are preferable, MEL-B and MEL-C are more preferable, and MEL-B is particularly preferable.

The biosurfactant of the present invention used as a cosmetic for skin care/skin roughness improvement can be produced by the fermentation of the microorganism as described above.

The amount of the biosurfactant added to the cosmetic varies depending on the type of the cosmetic to be targeted and cannot be defined collectively, but can be in the range in which the skin roughness improvement/skin care actions are not impaired. Typically, the amount is preferably 0.001 to 50% by mass, more preferably 0.1 to 20% by mass, still more preferably 1 to 15% by mass and particularly preferably 3 to 10% by mass relative to each cosmetic. Here, a use form of the biosurfactant added to the cosmetic is optional. For example, a biosurfactant extracted from a culture medium can be directly used, or a highly purified biosurfactant can be used, or a biosurfactant can be used after being suspended in water or dissolved in an organic solvent such as ethanol.

The biosurfactant may be combined in the cosmetic by dissolution in an oil-soluble base or an oil-soluble component, and is preferably combined in the form of a liposome in aqueous cosmetics such as face lotions and moisturizing liquids. The biosurfactant combined as the liposome is preferable because it is fused to skin cells to enhance its absorbability. The method to prepare the liposome is not particularly limited; any of the publicly known preparation methods, such as the ethanol injection method or the Bangham method, can be employed.

The method for producing the cosmetic of the present invention using the biosurfactant is not particularly limited, and the biosurfactant can be dissolved in a nonionic surfactant, lower alcohol, polyvalent alcohol, or natural fat or oil such as olive oil, squalane, a fatty acid or a higher alcohol.

Examples of nonionic surfactants include sorbitan fatty acid esters (e.g., sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, diglycerol sorbitan tetra-2-ethylhexylate); glycerine/polyglycerine fatty acids (e.g., mono-cottonseed oil fatty acid glycerine, glycerine monoerucate, glycerine sesquioleate, glycerine monostearate, glycerine α,α'-oleate pyroglutamate, glycerine monostearate malic acid); propylene glycol fatty acid esters (e.g., monostearic acid propylene glycol); cured castor oil derivatives; and glycerine alkyl ether.

Examples of POE-based hydrophilic nonionic surfactants include POE-sorbitan fatty acid esters (e.g., POE-sorbitan monooleate, POE-sorbitan monostearate, POE-sorbitan monooleate, POE-sorbitan tetraoleate); POE-sorbit fatty acid esters (e.g., POE-sorbit monolaurate, POE-sorbit monooleate, POE-sorbit pentaoleate, POE-sorbit monostearate); POE-glycerine fatty acid esters (e.g., POE-glycerine monostearate, POE-glycerine monoisostearate, POE-glycerine triisostearate, POE-monooleate); POE-fatty acid esters (e.g., POE-distearate, POE-monodioleate, distearic acid ethylene glycol); POE-alkyl ethers (e.g., POE-lauryl ether, POE-oleyl ether, POE-stearyl ether, POE-behenyl ether, POE-2-octyldodecyl ether, POE-cholestanol ether); Pluronic types (e.g., Pluronic); POE/POP-alkyl ethers (e.g., POE/POP-cetyl ether, POE/POP-2-decyltetradecyl ether, POE/POP-monobutyl ether, POE/POP-hydrogenated lanoline, POE/POP-glycerine ether); tetra-POE/tetra-POP-ethylenediamine condensates (e.g., Tetronic); POE-castor oil cured castor oil derivatives (e.g., POE-castor oil, POE-cured castor oil, POE-cured castor oil monoisostearate, POE-cured castor oil triisostearate, POE-cured castor oil pyroglutamate monoisostearate diester, POE-cured castor oil maleate); POE-beeswax lanoline derivatives (e.g., POE-sorbit beeswax); alkanol amide (e.g., palm oil fatty acid diethanolamide, lauric acid monoethanolamide, fatty acid isopropanolamide); POE-propylene glycol fatty acid ester; POE-alkylamine; POE-fatty acid amide; sucrose fatty acid ester; alkylethoxydimethylamine oxide; and trioleyl phosphate.

Examples of lower alcohols include ethanol, propanol, isopropanol, isobutyl alcohol and t-butyl alcohol.

Examples of polyvalent alcohols include bivalent alcohols (e.g., ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol); trivalent alcohols (e.g., glycerine, trimethylolpropane); tetravalent alcohols (e.g., pentaerythritols such as 1,2,6-hexanetriol); pentavalent alcohols (e.g., xylitol); hexavalent alcohols (e.g., sorbitol, mannitol); polyvalent alcohol polymers (e.g., diethylene glycol, dipropylene glycol, triethylene glycol, polypropylene glycol, tetraethylene glycol, diglycerine, polyethylene glycol, triglycerine, tetraglycerine, polyglycerine); bivalent alcohol alkyl ethers (e.g., ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether); bivalent alcohol alkyl ethers (e.g., diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether); bivalent alcohol ether esters (e.g., ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate); glycerine monoalkyl ethers (e.g., chimyl alcohol, selachyl alcohol, bathyl alcohol); sugars and sugar alcohols (e.g., sorbitol, maltitol, maltotriose, mannitol, sucrose, erythritol, glucose, fructose, starch-degraded sugar, maltose, xylitose, starch-degraded sugar-reduced alcohol); glysolid; tetrahydrofurfuryl alcohol; POE-tetrahydrofurfuryl alcohol; POP-butyl ether; POP/POE-butyl ether; tripolyoxypropylene glycerine ether; POP-glycerine ether; POP-glycerine ether phosphate; POP/POE-pentaerythritol ether, and polyglycerine.

Examples of oils include animal and plant oils such as avocado oil, olive oil, sesame oil, camellia oil, evening primrose oil, turtle oil, macadamia nut oil, corn oil, mink oil, rapeseed oil, egg yolk oil, parsic oil, wheat germ oil, sasanqua oil, castor oil, flaxseed oil, safflower oil, cotton seed oil, perilla oil, soybean oil, peanut oil, tea oil, kaya seed oil, rice bran oil, China wood oil, jojoba oil, cacao butter, fractionated coconut oil, horse oil, palm oil, palm kernel oil, beef tallow, mutton tallow, lard, lanoline, whale wax, beeswax, carnauba wax, vegetable wax, candelilla wax and squalane, cured oils thereof, mineral oils such as liquid paraffin and petrolatum, and synthetic triglycerines such as tripalmitate glycerine.

Examples of the fatty acid include lauric acid, myristic acid, palmitic acid, oleic acid, linoleic acid, linolenic acid, stearic acid, behenic acid, 12-hydroxystearic acid, isostearic acid, undecynoic acid, tolic acid, eicosapentaenoic acid and docosahexaenoic acid. Examples of the higher alcohol include lauryl alcohol, cetyl alcohol, stearyl alcohol, behenyl alcohol, myristyl alcohol, oleyl alcohol, cetostearyl alcohol, jojoba alcohol, lanoline alcohol, batyl alcohol, 2-decyltetradecanol, cholesterol, phytosterol and isostearyl alcohol. Examples of the synthetic ester include cetyl octanoate, octyldodecyl myristate, isopropyl myristate, myristyl myristate, isopropyl palmitate, butyl stearate, hexyl laurate, decyl oleate, dimethyloctanoic acid, cetyl lactate and myristyl lactate. Examples of the silicone include chain-shaped polysiloxanes such as dimethyl polysiloxane and methylphenyl polysiloxane, cyclic polysiloxanes such as decamethyl cyclopolysiloxane, and three-dimensional mesh structures of silicone resins.

The skin care cosmetics of the present invention include milky lotions, beauty liquids, creams, lotions, skin care oils, cleansing oils, bath oils, or facial washes, makeup removers, shampoos and body soaps.

The MELs of the present invention, particularly MEL-A, MEL-B and MEL-C, are more excellent than ceramide in terms of ease of use because they are easily produced and also have an emulsification action.

### EXAMPLES

The present invention will be described in more detail in the following Examples.

### (Method for evaluating action to improve skin roughness)

Tissues are taken out according to the main points of the handling manual accompanying the Test Skin LSE-002 or 003 kit (Toyobo Co., Ltd.). A ring for assuring a drug exposure site is adhered to an LSE tissue surface, and an aqueous solution of 0.1% sodium dodecyl sulfate (SDS) is added into the ring, which is then left to stand at room temperature for 5 minutes. Subsequently, SDS is removed using an aspirator, and 3 ml of an assay medium is sprayed using a pipette to wash the tissue. By these manipulations, moisturizing components in the horny layer were eluted, and a dry skin was made.

Subsequently, as test articles, each 80 µL of purified water or a cosmetic liquid (Fenatty cosmetic liquid, fatted; FANCL Corporation) was added on the LSE (living skin equivalent) tissue surface, which was then left to stand at room temperature for 60 minutes. Then, the test articles were aspirated and removed using the aspirator. Subsequently, the LSE tissue was placed on an assay tray on which an assay medium had not been placed, and incubated for 24 hours in a CO₂ incubator adjusted to a temperature of 37°C and a relative humidity of 15 to 20% RH. Then, the LSE tissue was removed from the CO₂ incubator, and 1.2 mL of a mixed solution of the assay medium containing 0.333 g/mL of a tetrazolium salt (MTT) reagent was added to the assay tray according to the main points of the handling manual accompanying the LSE-003 kit. The LSE tissue in the assay tray was incubated for 3 hours in a CO₂ incubator adjusted to a temperature of 37°C and a relative humidity of 15 to 20% RH.

After the treatment with MTT, a piece of the LSE tissue was obtained by punching out a central portion of the LSE tissue that included a polycarbonate film using a biopsy punch of 8 mmφ, then transferring it to a small test tube, and 700 µL of 0.04 N hydrochloric acid-isopropanol was added thereto to extract in a dark place for two hours. After termination of the extraction, the extracted solution was stirred and mixed thoroughly. Subsequently, an absorbance at 562 nm for extracted formazan having a blue-violet color was measured. The absorbance obtained by this method is closely associated with the effect of improving skin roughness. Thus, this method is effective for evaluating the skin roughness improvement quantitatively, simply and economically.

### Example 1: Production of MEL

One loop of *Pseudozyma antarctica* NBRC 10736 was inoculated in a seed medium (20 mL/500 mL Sakaguchi flask) to perform an inoculum culture. The culture was performed at 30°C overnight. The resulting culture medium was rendered in the inoculum. The seed medium was composed of 4% glucose, 0.3% NaNO₃, 0.02% MgSO₄·H₂O, 0.02% KH₂PO₄ and 0.1% yeast extract. The above inoculum (75 mL) was inoculated in 1.5 L (5 L-jar) of a production medium, and cultured at 30°C, 300 rpm (stirring frequency) and 0.5 L/min0 (air) using the 5 L-jar. A production medium was composed of 3% soybean oil, 0.02% MgSO₄·H₂O, 0.02% KH₂PO₄ and 0.1% yeast extract. The culture medium (250 mL) was centrifuged (6,500 rpm, 30 min), a supernatant was removed, and a precipitate (microbial cells) was collected. Ethyl acetate (50 mL) was added to the precipitate, which was then stirred thoroughly and centrifuged (8,500 rpm, 30 min) to separate the supernatant from the precipitate. The supernatant was concentrated using an evaporator. MEL fractions (MEL-A, MEL-B and MEL-C) were obtained by using silica gel and eluting with hexane : acetone =5:1 and hexane : acetone =1:2.

### Example 1A: Production of MEL-B

A frozen stock (0.2 mL) of *P. tsukubaensis* was inoculated in 20 mL of YM seed medium in a 500 mL Sakaguchi flask, and cultured at 26°C at 180 rpm overnight to make a seed inoculum. The seed inoculum was inoculated again in 20 mL of YM seed medium in a 500 mL Sakaguchi flask, and cultured at 26°C at 180 rpm overnight to make an inoculum. The inoculum (20 mL) was inoculated in 2 L of YM medium in a 5 L jar and cultured at 26°C at 300 rpm (1/4 VVM, 0.5 L air/min) for 8 days. The culture medium was centrifuged at 7,900 rpm at 4°C for 60 minutes to separate the microbial cells (including MEL-B) from the supernatant. Ethyl acetate (80 mL) was added to a microbial cell fraction, which was then shaken to be suspended thoroughly and then centrifuged at 7,900 rpm at 4°C for 30 minutes. An equivalent amount of brine was added to the resulting supernatant, and the mixture was stirred to yield an ethyl acetate layer. An appropriate amount of sodium sulfate anhydrate was added to the ethyl acetate layer, which was then left to stand for 30 minutes and evaporated to yield a crude product of purified MEL-B. The resulting crude product (20 g) of purified MEL-B was further purified by using a silica gel column (200 g) and eluting with hexane/acetone to yield a purified MEL-B product.

### Example 2

Although soybean oil was used as the production material in the production of MEL in Example 1, MEL-A, MEL-B and MEL-C are isolated and purified using olive oil as the production material instead, and cultured the same way as in Example 1. The MEL fractions obtained at this time are referred to as MEL-A (OL), MEL-B (OL) and MEL-C (OL), in order to distinguish them from the MEL obtained in Example 1.

### Example 3

Although soybean oil was used as the production material in the production of MEL in Example 1, MEL-A, MEL-B and MEL-C are isolated and purified using methyl myristate for the production material instead, and cultured the same way as in Example 1. The MEL fractions obtained at this time are referred to as MEL-A (MY), MEL-B (MY) and MEL-C (MY), in order to distinguish them from the MEL obtained in Example 1.

### Example 4: Evaluation of MEL-A in skin roughness model

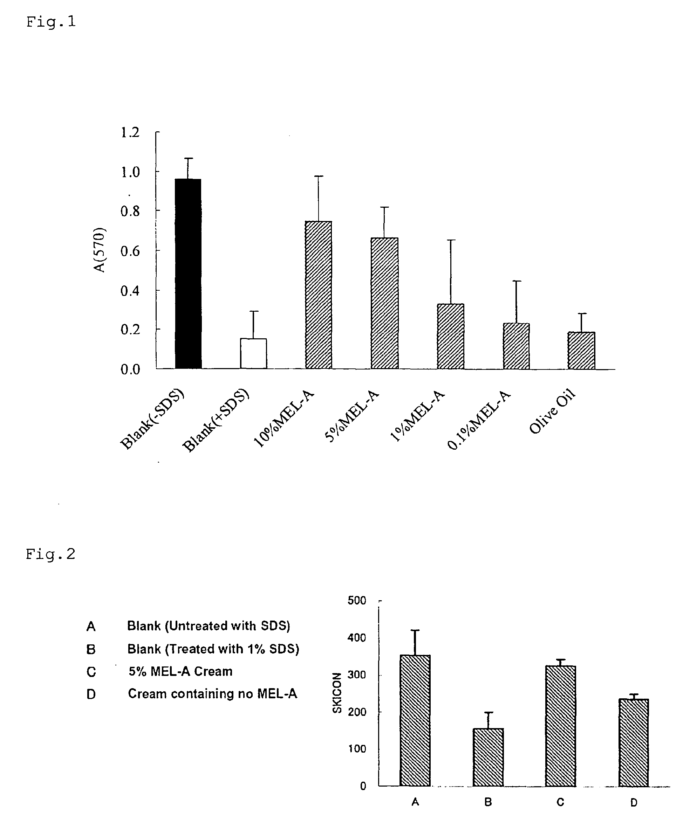

A skin roughness model using a three-dimensional skin model was made as follows. The skin roughness model was made by treating a Test Skin (LSE-002 or 003; Toyobo Co., Ltd.) with 1% SDS to remove the lipid components in the horny layer. The skin roughness prevention effect was examined by adding olive oil in which MEL-A was dissolved on the cells, leaving it to stand overnight, and subsequently calculating cell viability using a commercially available MTT kit. As shown in FIG. 1, the cell viability increased in a MEL-A-concentration-dependent manner, confirming that MEL-A acts as an alternative to ceramide. Meanwhile, the olive oil alone exhibited no such effect.

### Example 5: Evaluation of MEL-B in skin roughness model

The skin roughness model using the three-dimensional skin model was made as follows. The skin roughness model was made by treating a Test Skin (LSE-002 or 003; Toyobo Co., Ltd.) with 1% SDS to remove the lipid components in the horny layer. The skin roughness prevention effect was examined by adding the olive oil in which the MEL-B obtained in Example 1A was dissolved on the cells, leaving it to stand overnight, and subsequently calculating the cell viability using a commercially available MTT kit. As shown in FIG. 4, the cell viability increased in a MEL-B-concentration-dependent manner, confirming that MEL-B acts as an alternative to ceramide. Meanwhile, the olive oil alone exhibited no such effect.

### Example 6: Evaluation of MEL-C in skin roughness model

The skin roughness model using the three-dimensional skin model was made as follows. The skin roughness model was made by treating a Test Skin (LSE-002 or 003 supplied from Toyobo Co., Ltd.) with 1% SDS to remove the lipid components in the horny layer. The skin roughness prevention effect was examined by adding the olive oil in which the MEL-C obtained in Example 1 was dissolved on the cells, leaving it to stand overnight, and subsequently calculating the cell viability using a commercially available MTT kit. As shown in FIG. 4, the cell viability increased in a MEL-C-concentration-dependent manner, confirming that MEL-C acts as an alternative to ceramide. Meanwhile, the olive oil alone exhibited no such effect.

### Example 7: Evaluation of MEL-A (OL) and MEL-A (MY) in skin roughness model

Similar to Example 4, a skin roughness model using the three-dimensional skin model was made as follows. The skin roughness model was made by treating a Test Skin (LSE-002 or 003 supplied from Toyobo Co., Ltd.) with 1% SDS to remove the lipid components in the horny layer. The skin roughness prevention effect was examined by adding olive oil in which MEL-A (Example 1), MEL-A (OL) (Example 2) or MEL-A (MY) (Example 3) was dissolved on the cells, leaving it to stand overnight, and subsequently calculating the cell viability using a commercially available MTT kit. As shown in FIG. 3, the cell viability increased in a MEL-A-concentration-dependent manner, confirming that MEL-A acts as an alternative to ceramide. Meanwhile, the olive oil alone exhibited no such effect.

### Example 8: Effect of MEL-A-containing cream in human skin roughness test

The skin was roughened by placing an inner side of a human's upper arm in contact with a solution of 1% SDS for 10 minutes. Immediately following, the above cream containing 5% MEL-A was applied, and after 3 hours, the skin was washed with warm water. The oil content was wiped away with a Kim towel, and the moisture content of the skin's horny layer was measured using Skicon. As shown in FIG. 2, the recovery of the moisture content was observed when the MEL-A-containing cream was applied.

### Example 9: Effect of MEL-B-containing cream in human skin roughness test

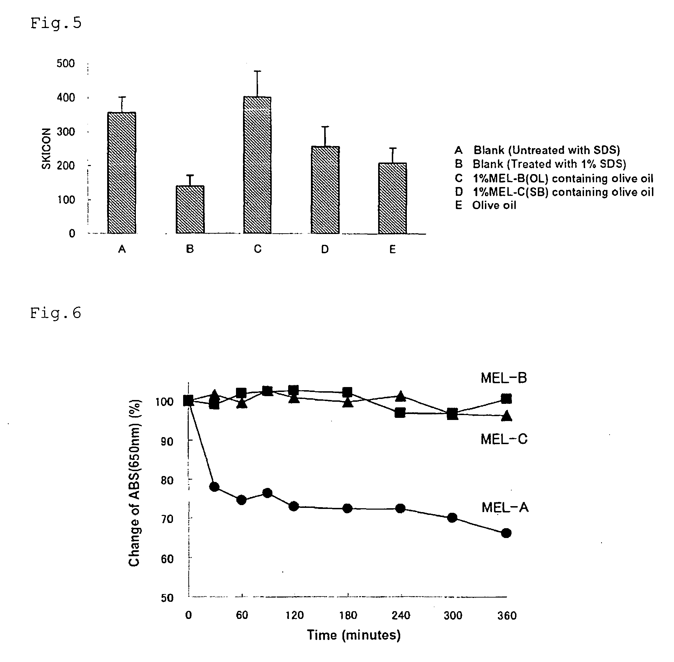

The skin was roughened by placing an inner side of a human's upper arm in contact with a solution of 1% SDS for 10 minutes. Immediately following, the above cream containing 5% MEL was applied, and after 3 hours, the skin was washed with warm water. The oil content was wiped away with Kim towel, and the moisture content of the skin's horny layer was measured using Skicon. As shown in FIG. 5, the recovery of the moisture content was observed when the MEL-B- or C-containing cream was applied.

### Example 10: Dispersion stability test of MEL-B and MEL-C

MEL-B or MEL-C at a concentration of 10 mg/mL was added to water, stirred and suspended. Its absorbance at 650 nm was measured. The results are shown in FIG. 6. From the results in FIG. 6, it is clear that MEL-B and MEL-C are particularly excellent in dispersion stability.

### Example 11: Preparation of a MEL-B liposome solution

A MEL-B liposome solution was prepared as follows using an ethanol injection method. 10 mg of MEL-B was dissolved in 0.5 mL of ethanol, and 1 mL of distilled water previously warmed to about 70°C was added. The mixture was slightly shaken and mixed, and residual ethanol was distilled off using a rotary evaporator. Sonication for about 5 minutes was applied thereto using a water bath-type sonicator (W-220R; Honda Electronics Co., Ltd.), and then the distilled water was added to make a total volume 1 mL.

A MEL-B liposome solution was prepared as follows using the Bangham method. 10 mg of MEL-B was dissolved in 1 mL of chloroform, and the solvent was distilled off using a rotary evaporator to make a thin film. Thereto, 1 mL of distilled water was added, and sonication for about 5 minutes was applied thereto using a water bath-type sonicator (W-220R; Honda Electronics Co., Ltd.).

A MEL-B suspension was prepared as follows. 1 mL of distilled water was added to 10 mg of MEL-B, and stirred using a vortex mixer to prepare the suspension.

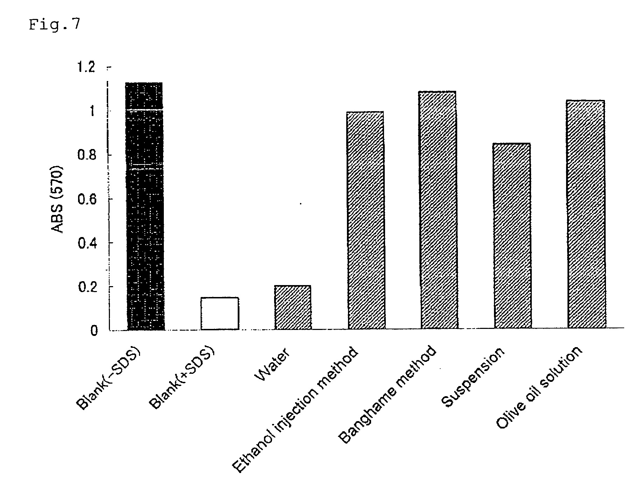

The MEL-B liposome aqueous solutions prepared by the ethanol injection method, Bangham method and MEL-B suspension were subjected to a skin roughness test using the three-dimensional skin model shown in Example 4, and their effects were examined. The results are shown in FIG. 7. As a result, the MEL-B liposome aqueous solution prepared by any of the ethanol injection method, Bangham method and the suspension exhibited the same skin roughness improvement effect as that of the MEL-B olive oil solution (MEL-B concentration: 1%).

### Example 12: Production of beauty liquid

A beauty liquid having the composition shown below was produced using standard methods. As a control, a MEL-free beauty liquid was also produced using standard methods.

| (Composition) | (% by weight) |
|---|---|
| Sorbit | 4.0 |
| Dipropylene glycol | 6.0 |
| Polyethylene glycol 1500 | 5.0 |
| POE (20) Oleyl alcohol ether | 0.5 |
| Sucrose fatty acid ester | 0.2 |
| methyl cellulose | 0.2 |
| MEL-B | 5.0 |
| Purified water | Amount to make the total 100% |

### Example 13: Production of milky lotion

A milky lotion having the composition shown below was produced using standard methods. As the control, a MEL-free milky lotion was also produced using standard methods.

| (Composition) | (% by weight) |
|---|---|
| Glyceryl ether | 1.5 |
| Sucrose fatty acid ester | 1.5 |
| Sorbitan monostearate | 1.0 |
| Squalane | 7.5 |
| Dipropylene glycol | 5.0 |
| MEL-B | 5.0 |
| Purified water | Amount to make the total 100% |

### Example 14: Production of cream

A cream having the composition shown below was produced using standard methods. As the control, a MEL-free cream was also produced using standard methods.

| (Composition) | (% by weight) |
|---|---|
| Propylene glycol | 6.0 |
| Dibutyl phthalate | 19.0 |
| Stearic acid | 5.0 |
| Glycerine monostearate | 5.0 |
| Sorbitan monostearate | 12.0 |
| Polyethylene sorbitan monostearate | 38.0 |
| Sodium edetate | 0.03 |
| MEL-B | 5.0 |
| Purified water | Amount to make the total 100% |

### Example 15: Production of oil for makeup

| (Composition) | (% by weight) |
|---|---|
| Olive oil | 90 |
| MEL-A | 10 |

### Example 16: Skin care oil

| (Composition) | (% by weight) |
|---|---|
| Olive oil | 50 |
| MEL-C | 30 |
| Squalane | 10 |
| Sesame oil | 10 |

### Example 17: Skin care oil

| (Composition) | (% by weight) |
|---|---|
| Olive oil | 39 |
| MEL-C | 59 |
| Sesame oil | 1 |
| Lavender oil | 0.4 |
| Rosemary oil | 0.4 |
| Sage oil | 0.1 |
| δ-Tocopherol | 0.1 |

### Example 18: Cleansing oil

| (Composition) | (% by weight) |
|---|---|
| Olive oil | 40 |
| MEL-B | 28 |
| Methylphenyl polysiloxane | 2 |
| Ethanol | 0.3 |
| Isostearic acid | 0.1 |
| Cetyl 2-ethylhexanoate | 20 |
| Polyethylene glycol diisostearate | 2 |
| Palm oil fatty acid diethanolamide | 0.1 |
| Polyethylene glycol monoisostearate | 2 |
| δ-Tocopherol | 0.1 |
| Purified water | 1 |
| Perfume | Appropriate quantity |

### Example 19: Bath oil

| (Composition) | (% by weight) |
|---|---|
| Olive oil | 25 |
| MEL-A | 25 |
| Liquid paraffin | 25 |
| Neopentyl glycol dicaprylate | 10 |
| Polyoxyethylene oleyl ether | 10 |
| Purified water | 0.5 |
| δ-Tocopherol | 0.1 |
| Perfume | Appropriate quantity |

### INDUSTRIAL APPLICABILITY

According to the present invention, it is possible to prevent skin roughness by supplementing removed ceramide with the MEL-A, MEL-B or MEL-C of the present invention, which can not only be used as emulsifiers, but can also be used as alternatives ceramide, as they are much more easily produced than ceramide and are thus expected to contribute greatly to the industry.

## Claims

1. Use of at least one biosurfactant for improving skin roughness, wherein the skin roughness is a skin in a dry state on which the exfoliation of corneocytes is observed, and wherein the biosurfactant is a mannosylerythritol lipid (MEL) and/or a mannosylmannitol lipid (MML).

2. The use according to claim 1, wherein the skin roughness is caused by an action of a surfactant.

3. The use according to claim 1 or 2, wherein the biosurfactant is at least one selected from the group consisting of mannosylerythritol lipid A (MEL-A), mannosylerythritol lipid B (MEL-B) and mannosylerythritol lipid C (MEL-C).

4. The use according to any one of claims 1 to 3, wherein the biosurfactant is mannosylerythritol lipid B (MEL-B).

5. The use according to any one of claims 1 to 3, wherein the biosurfactant is mannosylerythritol lipid C (MEL-C).

6. The use according to any one of claims 1 to 3, wherein the biosurfactant is mannosylerythritol lipid A (MEL-A).

7. The use according to any one of claims 1 to 6, wherein the biosurfactant is MEL produced by yeast belonging to Pseudozyma sp.

8. The use according to claim 7, wherein the yeast is Pseudozyma tsukubaensis.

## Patentansprüche

1. Verwendung von mindestens einem Biotensid zur Verbesserung der Hautrauigkeit, wobei die Hautrauigkeit eine Haut in einem trockenen Zustand ist, auf welcher die Abschuppung von Korneozyten beobachtet wird, und wobei das Biotensid ein Mannosylerythritollipid (MEL) und/oder ein Mannosylmannitollipid (MML) ist.

2. Verwendung nach Anspruch 1, wobei die Hautrauigkeit durch ein Einwirken eines Tensids verursacht wurde.

3. Verwendung nach Anspruch 1 oder 2, wobei das Biotensid mindestens eines ist, ausgewählt aus der Gruppe, bestehend aus Mannosylerythritollipid A (MEL-A), Mannosylerythritollipid B (MEL-B) und Mannosylerythritollipid C (MEL-C).

4. Verwendung nach einem der Ansprüche 1 bis 3, wobei das Biotensid Mannosylerythritollipid B (MEL-B) ist.

5. Verwendung nach einem der Ansprüche 1 bis 3, wobei das Biotensid Mannosylerythritollipid C (MEL-C) ist.

6. Verwendung nach einem der Ansprüche 1 bis 3, wobei das Biotensid Mannosylerythritollipid A (MEL-A) ist.

7. Verwendung nach einem der Ansprüche 1 bis 6, wobei das Biotensid MEL ist, hergestellt durch Hefe, die zu Pseudozyma sp gehört.

8. Verwendung nach Anspruch 7, wobei die Hefe Pseudozyma tsukubaensis ist.

## Revendications

1. Utilisation d'au moins un biotensioactif pour améliorer la peau rêche, dans laquelle la peau rêche est une peau à l'état sec sur laquelle on observe l'exfoliation des coméocytes, et dans laquelle le biotensioactif est un lipide mannosylérythritol (MEL) et/ou un lipide mannosylmannitol (MML).

2. Utilisation selon la revendication 1, dans laquelle la peau rêche est provoquée par une action d'un tensioactif.

3. Utilisation selon la revendication 1 ou 2, dans laquelle le biotensioactif est au moins l'un choisi dans le groupe constitué du lipide A mannosylérythritol (MEL-A), du lipide B mannosylérythritol (MEL-B) et du lipide C mannosylérythritol (MEL-C).

4. Utilisation selon l'une quelconque des revendications 1 à 3, dans laquelle le biotensioactif est le lipide B mannosylérythritol (MEL-B).

5. Utilisation selon l'une quelconque des revendications 1 à 3, dans laquelle le biotensioactif est le lipide C mannosylérythritol (MEL-C).

6. Utilisation selon l'une quelconque des revendications 1 à 3, dans laquelle le biotensioactif est le lipide A mannosylérythritol (MEL-A).

7. Utilisation selon l'une quelconque des revendications 1 à 6, dans laquelle le biotensioactif est le MEL produit par une levure appartement à Pseudozyma sp.

8. Utilisation selon la revendication 7, dans laquelle la levure est Pseudozyma tsukubaensis.
